(19) Europäisches Patentamt
European Patent Office
Office européen des brevets

(11) EP 0 519 548 B1

(12) **FASCICULE DE BREVET EUROPEEN**

(45) Date de publication et mention de la délivrance du brevet:
**23.04.1997 Bulletin 1997/17**

(51) Int. Cl.[6]: **B01J 31/02**, B01J 31/30, C07C 17/08

(21) Numéro de dépôt: **92201676.1**

(22) Date de dépôt: **10.06.1992**

# (54) Système catalytique d'hydrochloration et procédé de fabrication de chlorure de vinyle au départ d'acétylène et de chlorure d'hydrogène en présence de ce système catalytique

Hydrochlorierung katalytisches System und Verfahren zur Herstellung von Vinylchlorid aus Acetylen und Chlorwasserstoff, in Gegenwart dieses katalytischen Systems

Hydrochlorination catalyst system and preparation process of vinyl chloride from acetylène and hydrogen chloride in presence of this catalyst system

(84) Etats contractants désignés:
**AT BE DE ES FR GB GR IT NL PT SE**

(30) Priorité: **20.06.1991 BE 9100599**

(43) Date de publication de la demande:
**23.12.1992 Bulletin 1992/52**

(73) Titulaire: **SOLVAY (Société Anonyme)**
**B-1050 Bruxelles (BE)**

(72) Inventeurs:
- **Strebelle, Michel**
  **B-1150 Bruxelles (BE)**
- **Devos, André**
  **B-1940 Sint-Stevens-Woluwe (BE)**

(74) Mandataire: **Jacques, Philippe et al**
**Solvay S.A.**
**Département Propriété Industrielle**
**310, rue de Ransbeek**
**1120 Bruxelles (BE)**

(56) Documents cités:
**EP-A- 0 090 443**
**EP-A- 0 340 416**
**DE-A- 3 500 318**
**DE-C- 709 000**
**GB-A- 2 001 546**

- **CHEMICAL ABSTRACTS, vol. 71, no. 3, 1969, Columbus, Ohio, US; abstract no. 12510v, N. KARAPETYAN 'CATALYST FORSYNTHESIS OF VINYL CHLORIDE FROM ACETYLENE' page 269 ;**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.3/3.4

EP 0 519 548 B1

## Description

La présente invention concerne un système catalytique d'hydrochloration à base d'un composé de métal du groupe VIII et un procédé de fabrication de chlorure de vinyle par hydrochloration d'acétylène en présence d'un tel système catalytique.

La fabrication du chlorure de vinyle par réaction entre l'acétylène et le chlorure d'hydrogène est classiquement réalisée en phase gazeuse, dans un réacteur à lit fixe, en présence d'un catalyseur solide hétérogène à base de chlorure de mercure sur support. Principalement pour des raisons de toxicité, il existe actuellement un intérêt croissant pour des systèmes catalytiques exempts de composés du mercure. Divers catalyseurs destinés à remplacer les catalyseurs actuels dans les procédés en phase gazeuse ont été développés. Par exemple, la demande de brevet japonais non examinée 52/136104 décrit un procédé d'hydrochloration de l'acétylène en phase gazeuse en présence d'un lit fixe de catalyseur constitué d'halogénures de métaux nobles déposés sur charbon actif. Jusqu'à présent cependant, la durée de vie de tels catalyseurs alternatifs destinés à des procédés en phase gazeuse reste très inférieure à celle des catalyseurs à base de composés du mercure.

Il existe par ailleurs dans la littérature quelques exemples d'hydrochloration de l'acétylène en présence d'un milieu catalytique liquide. Le brevet allemand 709.000 décrit un procédé de préparation d'halogénures de vinyle par mise en contact à température élevée d'acétylène avec une masse fondue de sels d'halogénohydrates de bases organiques renfermant un catalyseur usuel. Comme bases organiques sont envisagées les amines aliphatiques, aromatiques ou hétérocycliques et leurs mélanges. Dans l'exemple 1, du chlorure de vinyle est obtenu par dispersion de chlorure d'hydrogène et d'acétylène dans un mélange constitué de 350 parties en volume de pyridine, de 350 parties en volume de diéthylamine et de 100 parties en poids de chlorure de mercure, maintenu à 220-225 °C. Le certificat d'auteur SU-237116 décrit l'emploi d'une solution aqueuse acide contenant 46 % en poids de chlorure cuivreux et de 14 à 16 % en poids d'un chlorhydrate de méthyl-, diméthyl- ou triméthylamine. La demande de brevet EP-A-0340416 divulgue un procédé de préparation du chlorure de vinyle par réaction d'acétylène avec du chlorure d'hydrogène en présence d'un composé de palladium comme catalyseur dans un solvant constitué par un amide aliphatique ou cycloaliphatique, à une température supérieure à la température ambiante. Bien qu'il permette d'atteindre des rendements élevés, ce procédé présente toutefois certains inconvénients importants : il est apparu que, dans les conditions de réaction, le système catalytique liquide se dégrade progressivement, en formant des produits noirâtres d'apparence charbonneuse. De plus, en présence de chlorure d'hydrogène, l'amide se transforme en chlorhydrate dont le point de fusion est généralement très supérieur à la température ambiante. Le chlorhydrate de N-méthyl-pyrrolidone, par exemple, n'est liquide qu'au-dessus de 80 °C. Dans la pratique, cela peut provoquer de sérieux problèmes de mise en oeuvre, problèmes liés à des phénomènes de prise en masse du milieu catalytique lors des arrêts du réacteur ou de bouchage des canalisations aux endroits les plus froids de l'installation. Tout le réacteur ainsi que les canalisations dans lesquelles circule le milieu réactionnel doivent dès lors être maintenus en permanence à une température supérieure à la température de fusion du chlorhydrate.

En conséquence, l'invention a pour but un système catalytique d'hydrochloration exempt de composés du mercure, stable et de mise en oeuvre aisée parce que restant liquide à température ambiante. L'invention a encore pour but de fournir un procédé de synthèse du chlorure de vinyle par hydrochloration de l'acétylène en présence d'un tel système catalytique qui ne se dégrade pas dans les conditions réactionnelles. A la différence des systèmes à base de composés du mercure, le système catalytique selon l'invention présente de plus l'avantage d'éviter la vaporisation de sels métalliques dans l'installation.

L'invention concerne un système catalytique d'hydrochloration, plus particulièrement d'hydrochloration de l'acétylène. Ce système catalytique comprend au moins un composé de métal du groupe VIII et un chlorhydrate d'amine dont le point de fusion est inférieur ou égal à 25 °C.

Des chlorhydrates d'amine dont le point de fusion est inférieur ou égal à 25 °C sont notamment les chlorhydrates d'amines à fort encombrement stérique, tels que les chlorhydrates d'amines répondant à la formule générique suivante :

$$\begin{array}{c} R1 \\ | \\ R3 - C - NH_2 \ . \ HCl \\ | \\ R2 \end{array} \qquad (I)$$

Dans cette formule, R1 et R2 sont des atomes d'hydrogène ou des groupements alkyle ou aryle identiques ou différents et R3 est un groupement alkyle ou aryle. Eventuellement R1 et R3 peuvent former ensemble, au moyen d'atomes de carbone les reliant, un cycle, par exemple à 5 ou 6 atomes de carbone, lesquels peuvent être substitués par des groupements alkyle. De préférence, R1, R2 et R3 sont des groupements alkyle.

Par groupement alkyle, on entend toute chaîne carbonée linéaire ou ramifiée, éventuellement substituée par un ou plusieurs groupements aryle. Par groupement aryle, on entend tout radical aromatique éventuellement substitué par un ou plusieurs groupements alkyles.

Le nombre total d'atomes de carbone dans ce composé est au moins égal à 8. Il est de préférence au moins égal à 12. Le nombre total d'atomes de carbone dans ce composé est généralement au plus égal à 30. Il est de préférence au plus égal à 24.

Par chlorhydrate d'amine, on entend désigner un ou plusieurs chlorhydrates d'amine, y compris tout mélange de chlorhydrates de plusieurs amines, par exemple, de plusieurs composés isomériques. Un tel mélange de chlorhydrates de plusieurs amines peut aussi être mis en oeuvre, notamment en raison de sa plus grande disponibilité ou de son plus faible coût par rapport aux composés purs. Un exemple d'un tel chlorhydrate d'amine comprenant un mélange de divers composés répondant à la formule (I) est obtenu, par réaction de chlorure d'hydrogène avec des produits commerciaux tels que les amines primaires tert-alkyle PRIMENE 81-R et PRIMENE JM-T de Rohm and Haas Co, constituées de mélanges d'amines isomériques respectivement en C12-C14 et en C18-C22. Dans certains cas, il peut aussi s'avérer intéressant de mélanger volontairement des chlorhydrates de différentes amines en raison de l'existence d'eutectiques entre ces composés, présentant un point de fusion inférieur à celui de chacun des constituants.

De bons résultats ont été obtenus avec un système catalytique comprenant un chlorhydrate d'amine tert-alkyle (R1, R2 et R3 représentant des groupements alkyles) contenant de 10 à 25 atomes de carbone tels que les amines primaires tert-alkyle PRIMENE 81-R et PRIMENE JM-T de Rohm and Haas Co. D'autres chlorhydrates d'amines ayant également donné de bons résultats sont les chlorhydrates d'amines dans lequels R1 et R2 sont des atomes d'hydrogène et R3 est un groupement aryle ou alkyle, par exemple le chlorhydrate de polyisopropylbenzylamine et le chlorhydrate de polyéthyl-β-phénéthylamine. De telles amines à encombrement stérique important peuvent être obtenues aisément, par exemple, au départ des amines correspondantes dont le noyau aromatique est non alkylé, - pour les composés ci-dessus, au départ respectivement de benzylamine et de 2-phényléthylamine - par protection de la fonction amine par réaction avec un anhydride d'acide carboxylique, alkylation classique du noyau aromatique de l'amide obtenu et enfin hydrolyse alcaline de la fonction amide.

Les composés de métaux du groupe VIII mis en oeuvre dans les systèmes catalytiques de la présente invention sont généralement choisis parmi les composés de fer, de cobalt, de nickel, de ruthénium, de rhodium, de palladium, d'osmium, d'iridium, de platine ou de leurs mélanges. Les chlorures de ces métaux du groupe VIII sont préférés, mais tout autre composé pouvant se transformer en chlorure en présence de chlorure d'hydrogène lors de la préparation du système catalytique peut aussi être utilisé. De préférence, le composé de métal du groupe VIII mis en oeuvre dans la présente invention est choisi parmi les composés de platine et les composés de palladium, tels que le chlorure de platine (II) ou le chlorure de palladium (II), un platinochlorure ou un palladochlorure des métaux alcalins ou des métaux alcalinoterreux - par exemple, $Na_2PtCl_4$, $Na_2PdCl_4$, $K_2PtCl_4$, $K_2PdCl_4$, $Li_2PtCl_4$, $Li_2PdCl_4$, $(NH4)_2PtCl_4$ et $(NH4)_2PdCl_4$ -, l'acide hexachloroplatinique ou ses sels, par exemple $Na_2PtCl_6$, $K_2PtCl_6$, $Li_2PtCl_6$, les composés de palladium dans lesquels le palladium a une valence élevée, comme $Na_2PdCl_6$, $K_2PdCl_6$, $Li_2PdCl_6$, etc. On peut aussi mettre en oeuvre des complexes des métaux du groupe VIII dans lesquel le métal est à la valence O, tels que les complexes $Pt(P\phi_3)_2$, $Pd(P\phi_3)_2$, $(P\phi_3)Pt(CO)$, etc.

Des mélanges de composés de métaux du groupe VIII peuvent aussi être utilisés.

Les composés de métaux du groupe VIII particulièrement préférés sont le chlorure de platine (II) et le chlorure de palladium (II). Lorsque le système catalytique contient un chlorhydrate d'amine tert-alkyle (R1, R2 et R3 représentant des groupes alkyles), le composé de métal du groupe VIII le plus particulièrement préféré est le chlorure de platine (II). Lorsque le système catalytique contient un chlorhydrate d'amine avec R1 et/ou R2 représentant un atome d'hydrogène, le composé de métal du groupe VIII le plus particulièrement préféré est le chlorure de palladium (II).

Le système catalytique le plus particulièrement préféré contient un chlorhydrate d'amine tert-alkyle, par exemple celui obtenu au départ de l'amine primaire tert-alkyle PRIMENE 81-R, et du chlorure de platine (II). Un tel système catalytique permet de synthétiser le chlorure de vinyle avec une sélectivité supérieure à 99,9 % et donc de diminuer fortement la quantité de sous-produits à éliminer. De plus, ce système ne se dégrade quasi pas au cours du temps.

La teneur en composé de métal du groupe VIII dans le système catalytique, exprimée en millimoles par litre de chlorhydrate d'amine est généralement supérieure ou égale à environ 1 mmol/l, de préférence supérieure ou égale à environ 10 mmol/l. La teneur en composé de métal du groupe VIII dans le système catalytique est généralement inférieure ou égale à environ 200 mmol/l, de préférence inférieure ou égale à environ 100 mmol/l. Bien que cela ne soit pas indispensable, il est cependant préférable que tout le composé de métal du groupe VIII compris dans le système catalytique soit sous forme dissoute.

L'invention concerne également un procédé de fabrication de chlorure de vinyle par hydrochloration de l'acétylène en présence d'un système catalytique comprenant au moins un composé de métal du groupe VIII et un chlorhydrate d'amine dont le point de fusion est inférieur ou égal à 25 °C. La nature et les proportions des constituants du système

catalytique mis en oeuvre dans le procédé selon l'invention sont celles définies ci-dessus.

Dans le procédé selon l'invention, le système catalytique défini ci-dessus peut être mis en oeuvre en phase liquide. Il peut aussi être déposé sur un support solide tel qu'une silice, une alumine ou un charbon actif, jusqu'à concurrence du volume poreux du support. De préférence, le système catalytique est mis en oeuvre en phase liquide. Cependant, la viscosité de ce liquide à la température de réaction limite souvent l'efficacité de l'échange de matières entre la phase gazeuse contenant les réactifs et la phase liquide dans laquelle se déroule la réaction d'hydrochloration. Dès lors, le système catalytique est de préférence dilué par un solvant organique. Le choix de la nature du solvant organique mis en oeuvre dans le procédé selon l'invention est conditionné notamment par la nécessité qu'il soit inerte vis-à-vis des réactifs dans les conditions de réaction, qu'il soit miscible avec le chlorhydrate d'amine et par le souhait qu'il forme avec ce chlorhydrate un milieu dont la viscosité est inférieure à celle du chlorhydrate seul. Par ailleurs, pour des raisons de sécurité et de facilité d'emploi, on donne la préférence aux solvants organiques peu volatils. Le choix du solvant organique est aussi influencé par sa capacité d'absorption de l'acétylène. Les solvants satisfaisant aux différents critères exposés ci-dessus sont choisis parmi les hydrocarbures aliphatiques, cycloaliphatiques et aromatiques et leurs mélanges, par exemple les paraffines en C7 à C15 et les alkylbenzènes, notamment les xylènes, les propylbenzènes, les butylbenzènes, les méthyléthylbenzènes. Pour des considérations de nature économique, le solvant mis en oeuvre est de préférence choisi parmi les produits commerciaux constitués de mélanges d'hydrocarbures aliphatiques tels que le solvant ISOPAR de Esso ou le solvant SHELLSOL K de Shell ou de mélanges de composés aromatiques tels que le solvant SOLVESSO de Esso ou le solvant SHELLSOL AB de Shell.

Des solvants ayant donné de bons résultats sont les solvants aliphatiques saturés, tels que le solvant SHELLSOL K constitué de coupes pétrolières ayant un point d'ébullition compris entre environ 190 °C et environ 250 °C.

D'autres solvants envisageables sur base des divers critères donnés ci-dessus sont certains composés halogénés lourds, tels que des halogénoalcanes, halogénobenzènes et autres dérivés halogénés de composés aromatiques.

Lorsque le système catalytique est mis en oeuvre en phase liquide et qu'il est dilué par un solvant organique, le rapport pondéral entre le solvant et le chlorhydrate d'amine est généralement supérieur ou égal à environ 0,01. De préférence, ce rapport est supérieur ou égal à environ 0,05. Dans les conditions particulièrement préférées, il est supérieur ou égal à environ 0,2. Ce rapport est généralement inférieur ou égal à environ 5. De préférence, il est inférieur ou égal à environ 3. Dans les conditions particulièrement préférées, il est inférieur ou égal à environ 2.

Le procédé selon l'invention est réalisable de la température ambiante jusqu'à environ 220 °C. A plus haute température, le système catalytique a tendance à se dégrader rapidement. La température de réaction préférée, c'est-à-dire celle offrant le meilleur compromis entre productivité, rendement et stabilité du milieu catalytique est supérieure ou égale à environ 80 °C. Les meilleurs résultats sont obtenus à des températures supérieures ou égales à environ 120 °C. De préférence, la température de réaction ne dépasse pas environ 200 °C. Une température de réaction inférieure ou égale à environ 170 °C est particulièrement préférée. Le procédé selon l'invention est généralement effectué à la pression atmosphérique ou à une pression légèrement supérieure compatible avec les règles de sécurité de manipulation de l'acétylène, c'est-à-dire ne dépassant pas environ 1,5 bar.

Le procédé de fabrication de chlorure de vinyle par hydrochloration de l'acétylène selon l'invention est réalisé par mise en contact dans tout réacteur approprié, des réactifs gazeux - acétylène et chlorure d'hydrogène - avec le système catalytique.

Lorsque le système catalytique est mis en oeuvre en phase liquide, le procédé selon l'invention peut être réalisé classiquement dans tout appareillage favorisant l'échange gaz-liquide, tel qu'une colonne à plateaux ou une colonne noyée à empilages. Un autre mode de mise en oeuvre du procédé permettant de bons échanges de matière entre les phases liquide et gazeuse consiste en l'emploi d'un réacteur à contre-courant, éventuellement du type empilages à lit arrosé, le système catalytique liquide ruisselant sur les empilages, à contre-courant du flux gazeux des réactifs.

Lorsque le système catalytique est déposé sur un support solide approprié, il peut avantageusement remplacer les catalyseurs au mercure dans les installations actuelles opérant avec des réacteurs à lit fixe.

Dans le procédé selon l'invention, le rapport molaire entre le chlorure d'hydrogène et l'acétylène introduits dans le réacteur est en général supérieur ou égal à environ 0,5. De préférence, ce rapport est supérieur ou égal à environ 0,8. En général, ce rapport molaire est inférieur ou égal à environ 3. De bons résultats ont été obtenus avec un rapport molaire entre le chlorure d'hydrogène et l'acétylène introduits dans le réacteur inférieur ou égal à environ 1,5. L'acétylène et le chlorure d'hydrogène peuvent être mis en contact dans le réacteur ou, de préférence, mélangés préalablement à leur introduction dans le réacteur.

Lorsqu'on opère en milieu liquide, dans le but d'augmenter la quantité d'acétylène dissous dans la phase liquide, il est également possible de mettre en oeuvre un procédé dans lequel seul l'acétylène est introduit dans le réacteur sous forme gazeuse, où il réagit avec le chlorure d'hydrogène présent dans la phase liquide sous forme de chlorhydrate, le chlorhydrate d'amine du système catalytique étant régénéré par mise en contact d'une navette liquide renfermant l'amine avec du chlorure d'hydrogène en dehors du réacteur.

Généralement, le système catalytique est préparé par dissolution ou dispersion de la quantité voulue de composé de métal du groupe VIII dans l'amine ou dans le mélange amine/solvant organique, puis par saturation de cette solution par du chlorure d'hydrogène provoquant la formation du chlorhydrate d'amine. Il est cependant également possible de,

d'abord saturer l'amine ou le mélange amine/solvant organique avec du chlorure d'hydrogène afin de former le chlorhydrate d'amine, puis d'introduire ensuite le composé de métal du groupe VIII dans le chlorhydrate d'amine ou dans le mélange de celui-ci avec le solvant organique. Habituellement, la quantité de composé de métal du groupe VIII mise en oeuvre est telle que, dans le système catalytique, tout le composé de métal du groupe VIII se trouve sous forme dissoute. A titre indicatif, la solubilité du chlorure de platine (II) dans le mélange en parts égales en poids de chlorhydrate de l'amine PRIMENE 81-R et de solvant SHELLSOL K dépasse 1 mol/l. On peut cependant aussi mettre en oeuvre un composé de métal du groupe VIII en quantité ou de nature telle qu'une fraction au moins de ce composé soit présente dans le système catalytique sous forme solide dispersé, sans porter préjudice à l'invention.

L'invention est illustrée par les exemples suivants. Les exemples 1 à 5, 7, 13 à 15 et 18 à 27 sont réalisés selon l'invention. Les exemples 6(C), 8(C) à 12(C), 16(C) et 17(C) sont réalisés à titre de comparaison.

Exemples 1 à 6(C)

Le système catalytique est préparé au départ d'amine PRIMENE 81-R, de chlorure de palladium et éventuellement de solvant SHELLSOL K.

L'amine PRIMENE 81-R est une amine primaire tert-alkyle, commercialisée par Rohm and Haas. Il s'agit d'un mélange d'amines, dont le nombre d'atomes de carbone est de 12 à 14. Le solvant SHELLSOL K, commercialisé par Shell, est constitué d'un mélange d'hydrocarbures, essentiellement de nature aliphatique. Le solvant utilisé dans ces exemples a un point d'ébullition initial de 193 °C et un point d'ébullition final de 245 °C.

L'amine PRIMENE 81-R est d'abord mélangée à des quantités variables de solvant SHELLSOL K, puis 4 g de chlorure de palladium (II), soit 22,6 mmol, sont introduits sous agitation dans un litre de solution. Le système catalytique est ensuite préparé par saturation de la solution par du chlorure d'hydrogène gazeux.

La réaction entre acétylène et chlorure d'hydrogène est réalisée de la manière suivante :

Un réacteur en pyrex de volume interne de 45 ml, muni d'une double enveloppe dans laquelle circule une huile caloporteuse et d'un dispositif d'introduction des réactifs constitué d'un embout en verre fritté destiné à assurer la dispersion des gaz dans le milieu liquide est chargé avec 30 ml d'une solution constituée par l'amine PRIMENE 81-R, le chlorure de palladium (II) et, éventuellement, le solvant SHELLSOL K.

La solution est chauffée à 150 °C et un flux gazeux renfermant un mélange de chlorure d'hydrogène et d'acétylène avec un rapport molaire $HCl/C_2H_2$ de 1,17 est introduit dans le réacteur. Selon les essais, le temps de séjour des gaz dans le réacteur, c'est-à-dire le rapport entre le volume du réacteur et le débit volumique des réactifs à la température de réaction est de 2,5 s ou de 4,9 s. Le produit gazeux sortant du réacteur est analysé par chromatographie en phase gazeuse. Les seuls produits de réaction observés sont le chlorure de (VC) et le 1-chloroprène (1CPr). Les résultats sont rassemblés au tableau I. La quantité de chlorure de vinyle produit est exprimée en grammes par heure et par litre de système catalytique. La sélectivité est définie comme le rapport molaire entre le VC produit et la somme [ VC + (2 x 1CPr) ].

TABLEAU I

| N° Ex. | Rapport pondéral amine PRIMENE 81R / solvent SHELLSOL K | Temps de séjour (s) | VC produit ($g.h^{-1}.l^{-1}$) | Sélectivité (%) | Remarques |
|---|---|---|---|---|---|
| 1 | 100 / 0 | 2,5 | 44 | 91,7 | |
| 2 | 75 / 25 | 4,9 | 126 | 93,4 | |
| 3 | 60 / 40 | 4,9 | 122 | 92,7 | |
| 4 | 50 / 50 | 4,9 | 100 | 91,6 | |
| 5 | 25 / 75 | 4,9 | 47 | 90,6 | |
| 6(C) | 0 / 100 | 4,9 | 2 | n.d. | Dégradation du milieu réactionnel |

Exemples 7 à 12(C)

Divers systèmes catalytiques sont préparés de la même manière que dans l'exemple 4, (quantités pondérales identiques d'amine PRIMENE 81-R et de solvant SHELLSOL K), mais le chlorure de palladium est remplacé par une quantité molaire identique (22,6 mmol/l) de chlorure de platine (II), de chlorure de cuivre (I), de chlorure de cuivre (II), de chlorure de zinc (II), de chlorure de bismuth (III) ou de chlorure d'étain (II).

La réaction d'hydrochloration de l'acétylène est effectuée dans les mêmes conditions qu'aux exemples 2 à 6, à

l'exception du temps de séjour égal à 4,95 s. Les résultats sont rassemblés au tableau II.

TABLEAU II

| N° exemple | Nature du composé métallique | VC produit ($g.h^{-1}.l^{-1}$) | Sélectivité (%) | Remarques |
|---|---|---|---|---|
| 7 | $PtCl_2$ | 270 | 100 | Solubilité totale |
| 8(C) | CuCl | 0 | n.d. | Solubilité totale |
| 9(C) | $CuCl_2$ | 0 | n.d. | Solubilité quasi totale |
| 10(C) | $ZnCl_2$ | 3 | n.d. | Solubilité totale |
| 11(C) | $BiCl_3$ | 3 | n.d. | Solubilité partielle |
| 12(C) | $SnCl_2$ | 0.6 | n.d. | Solubilité partielle |

Exemples 13 à 17(C)

Divers systèmes catalytiques sont préparés de la même manière que dans les exemples 4 ou 7, avec des quantités pondérales identiques d'amine et de solvant SHELLSOL K, mais l'amine PRIMENE 81-R est remplacée par d'autres composés azotés. L'amine PRIMENE JM-T mise en oeuvre dans l'essai 13 est un mélange d'amines primaires tert-alkyle en C18-C22, commercialisée également par Rohm and Haas.

La réaction d'hydrochloration de l'acétylène est effectuée dans les mêmes conditions de température et de proportions des réactifs qu'aux exemples 4 et 7. Dans les exemples 13 à 16 (c), le temps de séjour est de 4,9 s; il est de 2,5 s dans l'essai 17 (c). Les résultats sont rassemblés au tableau III.

TABLEAU III

| N° exemple | Nature du composé azoté | Nature du composé métallique | VC produit ($g.h^{-1}.l^{-1}$) | Sélectivité (%) | Remarques |
|---|---|---|---|---|---|
| 13 | amine PRIMENE JM-T | $PdCl_2$ | 86 | 92,2 | |
| 14 | mélange 1:3 en poids de polyisopropyl benzylamine et de polyéthyl-β-phénéthylamine | $PdCl_2$ | 320 | 97,6 | |
| 15 | mélange 1:1 en poids de polyisopropyl benzylamine et de polyéthyl-β-phén éthyl-amine | $PtCl_2$ | 35 | 99 | |
| 16(C) | Diméthyl-formamide | $PdCl_2$ | 116 | 93,2 | Dégradation du milieu réactionnel |
| 17(C) | N-méthyl-pyrrolidone | $PdCl_2$ | 243 | 98,7 | Système catalytique solide à temp. ≤ 80 °C |

Exemples 18 à 21

Divers systèmes catalytiques sont préparés de la même manière que dans l'exemple 7 (quantités pondérales identiques d'amine PRIMENE 81-R et de solvant SHELLSOL K), mais avec des quantités variables de chlorure de platine (II).

La réaction d'hydrochloration de l'acétylène est effectuée dans les mêmes conditions qu'à l'exemple 7. Les résultats sont rassemblés au tableau IV. Le rendement est défini comme le rapport molaire entre le VC produit et l'acétylène mis en oeuvre.

TABLEAU IV

| N° essai | Concentration en $PtCl_2$ (mmol/l) | Rendement (%) | VC produit ($g.h^{-1}.l^{-1}$) | Sélectivité (%) |
|---|---|---|---|---|
| 18 | 11,3 | 18 | 110 | 99,2 |
| 19 | 15 | 27,5 | 167 | 99,8 |
| 20 | 22,6 | 43 | 262 | 99,9 |
| 21 | 45,1 | 77,3 | 471 | 99,9 |

Exemples 22 à 25

Le système catalytique de l'exemple 20 est mis en oeuvre dans un réacteur micropilote, de même principe que le réacteur utilisé dans les exemples 1 à 21, mais constitué d'une colonne en pyrex de 2 m de haut et de 2,54 cm de diamètre, garni d'empilages de type anneaux de Rashig. Il est chargé avec 500 ml de solution catalytique.

La réaction d'hydrochloration de l'acétylène est effectuée dans les mêmes conditions opératoires de température et de proportions des réactifs que dans les exemples précédents, mais à différents temps de séjour. Les résultats sont rassemblés au tableau V.

TABLEAU V

| N° exemple | Temps de séjour (s) | Rendement (%) | VC produit ($g.h^{-1}.l^{-1}$) | Sélectivité (%) |
|---|---|---|---|---|
| 22 | 4,9 | 17,6 | 107 | 99,94 |
| 23 | 9,7 | 41 | 126 | 99,93 |
| 24 | 19,8 | 79,7 | 120 | 99,95 |
| 25 | 38,4 | 98,8 | 77 | 99,97 |

Exemple 26

Divers systèmes catalytiques sont préparés de la même manière que dans l'exemple 20 mais le solvant SHELLSOL K est remplacé par du solvant SOLVESSO 150. Le solvant SOLVESSO 150, commercialisé par Esso, est constitué d'un mélange d'hydrocarbures, essentiellement de nature aromatique (alkylbenzènes), dont le point initial d'ébullition est de 188 °C et le point final d'ébullition est de 208 °C. La réaction d'hydrochloration de l'acétylène est effectuée dans les mêmes conditions qu'à l'exemple 20. Les résultats sont rassemblés au tableau VI.

TABLEAU VI

| N° exemple | Temps de séjour (s) | Rendement (%) | VC produit ($g.h^{-1}.l^{-1}$) | Sélectivité (%) |
|---|---|---|---|---|
| 24 | 4,95 | 44,5 | 271 | 99,95 |

Exemple 27

Le système catalytique est préparé par imprégnation de 50 ml d'une alumine, préalablement séchée 24 heures à 250 °C, par 19,4 ml d'une solution d'amine PRIMENE 81-R contenant 22,6 mmole de $PtCl_2$ par litre, puis par passage d'un courant de chlorure d'hydrogène gazeux sur l'alumine imprégnée, placée dans le réacteur. L'alumine utilisée est une alumine ALUPERL KC GS 2078/3 de KALI CHEMIE. Ses caractéristiques sont les suivantes : billes de 3 à 4 mm de diamètre; surface spécifique BET : 179 $m^2/g$; volume poreux : 0,63 ml/g; poids spécifique : 0,71 g/ml; volume d'eau d'absorption : 0,59 ml/g. On utilise un réacteur similaire à celui utilisé dans les exemples 1 à 21, mais de volume interne égal à 70 ml. Le réacteur est chauffé à 200 °C et un flux gazeux renfermant un mélange de chlorure d'hydrogène et

d'acétylène avec un rapport molaire $HCl/C_2H_2$ de 1,17 est introduit dans le réacteur. Le temps de séjour des gaz est de 7,5 s. La quantité de chlorure de vinyle produit est de 32,2 grammes par heure et par gramme de platine mis en oeuvre. La sélectivité de la réaction est de 99,71 %.

## Revendications

1. Système catalytique d'hydrochloration comprenant au moins un composé de métal du groupe VIII et un chlorhydrate d'amine dont le point de fusion est inférieur ou égal à 25 °C.

2. Système catalytique selon la revendication 1 caractérisé en ce que le chlorhydrate d'amine répond à la formule

$$\begin{array}{c} R1 \\ | \\ R3 - C - NH_2 \cdot HCl \\ | \\ R2 \end{array}$$

avec R1 et R2 représentant des atomes d'hydrogène ou des groupements alkyle ou aryle identiques ou différents et R3 un groupement alkyle ou aryle, ledit chlorhydrate d'amine contenant de 8 à 30 atomes de carbone.

3. Système catalytique selon une quelconque des revendications 1 à 2 caractérisé en ce que le composé de métal du groupe VIII est choisi parmi les composés de palladium et les composés de platine.

4. Système catalytique selon une quelconque des revendications 1 à 3 caractérisé en ce que la teneur en composé de métal du groupe VIII exprimée en millimoles par litre de chlorhydrate d'amine est supérieure ou égale à environ 1 mmol/l et inférieure ou égale à environ 200 mmol/l.

5. Procédé de fabrication de chlorure de vinyle par réaction de l'acétylène avec du chlorure d'hydrogène à une température allant de la température ambiante jusqu'à environ 220 °C en présence d'un système catalytique caractérisé en ce que le système catalytique comprend au moins un composé de métal du groupe VIII et un chlorhydrate d'amine dont le point de fusion est inférieur ou égal à 25 °C.

6. Procédé selon la revendication 5 caractérisé en ce que le chlorhydrate d'amine répond à la formule

$$\begin{array}{c} R1 \\ | \\ R3 - C - NH_2 \cdot HCl \\ | \\ R2 \end{array}$$

avec R1 et R2 représentant des atomes d'hydrogène ou des groupements alkyle ou aryle identiques ou différents et R3 un groupement alkyle ou aryle, ledit chlorhydrate d'amine contenant de 8 à 30 atomes de carbone.

7. Procédé selon une quelconque des revendications 5 ou 6 caractérisé en ce que le composé de métal du groupe VIII est choisi parmi les composés de palladium et les composés de platine.

8. Procédé selon une quelconque des revendications 5 à 7 caractérisé en ce que la teneur en composé de métal du groupe VIII exprimée en millimoles par litre de chlorhydrate d'amine est supérieure ou égale à environ 1 mmol/l et inférieure ou égale à environ 200 mmol/l.

9. Procédé selon une quelconque des revendications 5 à 8 caractérisé en ce que le système catalytique est déposé sur un support solide.

**10.** Procédé selon une quelconque des revendications 5 à 8 caractérisé en ce que le système catalytique est mis en oeuvre en phase liquide.

**11.** Procédé selon la revendication 10 caractérisé en ce que le système catalytique est dilué par un solvant organique choisi parmi les hydrocarbures aliphatiques, cycloaliphatiques et aromatiques et leurs mélanges et en ce que le rapport pondéral entre ledit solvant et le chlorhydrate d'amine varie d'environ 0,01 à environ 5.

**12.** Procédé selon une quelconque des revendications 5 à 11 caractérisé en ce que la réaction est effectuée à une température d'environ 80 °C à environ 200 °C.

**13.** Procédé selon une quelconque des revendications 5 à 12 caractérisé en ce que le chlorure d'hydrogène et l'acétylène sont mis en oeuvre dans un rapport molaire d'environ 0,5 à environ 3.

## Claims

**1.** Catalytic hydrochlorination system comprising at least one group VIII metal compound and an amine hydrochloride whose melting point is lower than or equal to 25°C.

**2.** Catalytic system according to Claim 1 characterised in that the amine hydrochloride corresponds to the formula

$$R3 - \underset{\underset{R2}{|}}{\overset{\overset{R1}{|}}{C}} - NH_2 \cdot HCl$$

with R1 and R2 representing hydrogen atoms or optionally identical alkyl or aryl groups and R3 an alkyl or aryl group, the said amine hydrochloride containing from 8 to 30 carbon atoms.

**3.** Catalytic system according to any one of Claims 1 to 2, characterised in that the group VIII metal compound is chosen from palladium compounds and platinum compounds.

**4.** Catalytic system according to any one of Claims 1 to 3, characterised in that the content of group VIII metal compound expressed in millimoles per litre of amine hydrochloride is greater than or equal to approximately 1 mmol/l and less than or equal to approximately 200 mmol/l.

**5.** Process for the manufacture of vinyl chloride by reacting acetylene with hydrogen chloride at a temperature from room temperature to approximately 220°C in the presence of a catalytic system, characterised in that the catalytic system comprises at least one group VIII metal compound and an amine hydrochloride whose melting point is lower than or equal to 25°C.

**6.** Process according to Claim 5, characterised in that the amine hydrochloride corresponds to the formula

$$R3 - \underset{\underset{R2}{|}}{\overset{\overset{R1}{|}}{C}} - NH_2 \cdot HCl$$

with R1 and R2 representing hydrogen atoms or optionally identical alkyl or aryl groups and R3 an alkyl or aryl group, the said amine hydrochloride containing from 8 to 30 carbon atoms.

7. Process according to any one of Claims 5 or 6, characterised in that the group VIII metal compound is chosen from palladium compounds and platinum compounds.

8. Process according to any one of Claims 5 to 7, characterised in that the content of group VIII metal compound expressed in millimoles per litre of amine hydrochloride is greater than or equal to approximately 1 mmol/l and less than or equal to approximately 200 mmol/l.

9. Process according to any one of Claims 5 to 8, characterised in that the catalytic system is deposited on a solid support.

10. Process according to any one of Claims 5 to 8, characterised in that the catalytic system is used in the liquid phase.

11. Process according to Claim 10, characterised in that the catalytic system is diluted with an organic solvent chosen from aliphatic, cycloaliphatic and aromatic hydrocarbons and their mixtures and in that the weight ratio between the said solvent and the amine hydrochloride varies from approximately 0.01 to approximately 5.

12. Process according to any one of Claims 5 to 11, characterised in that the reaction is carried out at a temperature from approximately 80°C to approximately 200°C.

13. Process according to any one of Claims 5 to 12, characterised in that the hydrogen chloride and the acetylene are used in a molar ratio of approximately 0.5 to approximately 3.

**Patentansprüche**

1. Katalytisches System zur Hydrochlorierung, das wenigstens eine Verbindung eines Metalls der Gruppe VIII und ein Aminhydrochlorid, dessen Schmelzpunkt kleiner oder gleich 25 °C ist, umfaßt.

2. Katalytisches System gemäß Anspruch 1, dadurch gekennzeichnet, daß das Aminhydrochlorid der Formel

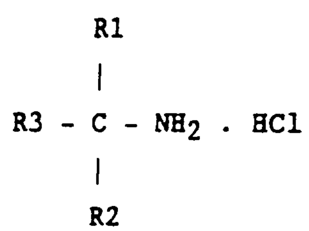

in der R1 und R2 Wasserstoffatome oder gleiche oder verschiedene Alkyl- oder Arylgruppen und R3 eine Alkyl- oder Arylgruppe darstellen, entspricht, wobei das besagte Aminhydrochlorid 8 bis 30 Kohlenstoffatome enthält.

3. Katalytisches System gemäß einem der Ansprüche 1 bis 2, dadurch gekennzeichnet, daß die Verbindung des Metalls der Gruppe VIII unter den Palladiumverbindungen und Platinverbindungen ausgewählt ist.

4. Katalytisches System gemäß einem der Ansprüche 1 bis 3, dadurch gekennzeichnet, daß der Gehalt an Verbindung des Metalls der Gruppe VIII, ausgedrückt in Millimol pro Liter Aminhydrochlorid, größer oder gleich etwa 1 mmol/l und kleiner oder gleich etwa 200 mmol/l ist.

5. Verfahren zur Herstellung von Vinylchlorid durch Reaktion von Acetylen mit Chlorwasserstoff bei einer Temperatur, die von der Raumtemperatur bis zu etwa 220 °C geht, in Gegenwart eines katalytischen Systems, dadurch gekennzeichnet, daß das katalytische System wenigstens eine Verbindung eines Metalls der Gruppe VIII und ein Aminhydrochlorid, dessen Schmelzpunkt kleiner oder gleich 25 °C ist, umfaßt.

6. Verfahren gemäß Anspruch 5, dadurch gekennzeichnet, daß das Aminhydrochlorid der Formel

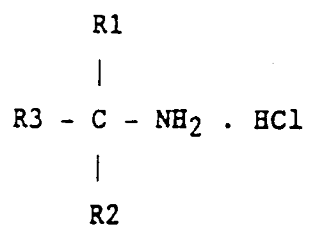

in der R1 und R2 Wasserstoffatome oder gleiche oder verschiedene Alkyl- oder Arylgruppen und R3 eine Alkyl- oder Arylgruppe darstellen, entspricht, wobei das besagte Aminhydrochlorid 8 bis 30 Kohlenstoffatome enthält.

7. Verfahren gemäß einem der Ansprüche 5 oder 6, dadurch gekennzeichnet, daß die Verbindung des Metalls der Gruppe VIII unter den Palladiumverbindungen und Platinverbindungen ausgewählt ist.

8. Verfahren gemäß einem der Ansprüche 5 bis 7, dadurch gekennzeichnet daß der Gehalt an Verbindung des Metalls der Gruppe VIII, ausgedrückt in Millimol pro Liter Aminhydrochlorid, größer oder gleich etwa 1 mmol/l und kleiner oder gleich etwa 200 mmol/l ist.

9. Verfahren gemäß einem der Ansprüche 5 bis 8, dadurch gekennzeichnet, daß das katalytische System auf einem festen Träger abgeschieden ist.

10. Verfahren gemäß einem der Ansprüche 5 bis 8, dadurch gekennzeichnet daß das katalytische System in flüssiger Phase verwendet wird.

11. Verfahren gemäß Anspruch 10, dadurch gekennzeichnet, daß das katalytische System durch ein organisches Lösungsmittel, das unter den aliphatischen, cycloaliphatischen und aromatischen Kohlenwasserstoffen und ihren Gemischen ausgewählt ist, verdünnt wird und dadurch, daß das Gewichtsverhältnis zwischen besagtem Lösungsmittel und dem Aminhydrochlorid von etwa 0,01 bis etwa 5 variiert.

12. Verfahren gemäß einem der Ansprüche 5 bis 11, dadurch gekennzeichnet, daß die Reaktion bei einer Temperatur von etwa 80 °C bis etwa 200 °C ausgeführt wird.

13. Verfahren gemäß einem der Ansprüche 5 bis 12, dadurch gekennzeichnet, daß Chlorwasserstoff und Acetylen in einem Molverhältnis von etwa 0,5 bis etwa 3 verwendet werden.